# EUROPÄISCHE PATENTSCHRIFT

(11) **EP 0 931 549 B1**
(45) Veröffentlichungstag und Bekanntmachung des Hinweises auf die Patenterteilung: **28.02.2007**
(21) Anmeldenummer: 98120471.2
(22) Anmeldetag: 29.10.1998
(51) Int. Cl.: A61K 36/48, A61P 19/10

(54) **Calcium/Soja-Instantgranulat**
Calcium/soy instant granulate
Granule instantané contenant du calcium et du soja

(30) Priorität: 29.10.1997 CH 250597; 29.10.1997 US 63674 P
(43) Veröffentlichungstag der Anmeldung: 28.07.1999
(73) Patentinhaber: Dr. Gergely & Co., 1053 Wien (AT)
(72) Erfinder: Gergely, Gerhard, Dr., 1053 Wien (AT); Gergely, Thomas, Dr., 1053 Wien (AT); Gergely, Irmgard, 1053 Wien (AT); Gergely, Stefan, Dr., 1053 Wien (AT)
(74) Vertreter: Bogensberger, Burkhard

(56) Entgegenhaltungen:
- EP-A- 0 398 867
- US-A- 5 424 331
- US-A- 5 654 011
- DATABASE WPI Section Ch, Week 9750 Derwent Publications Ltd., London, GB; Class D13, AN 97-536791 XP002069253 & CN 1 128 108 A (TONG K)

## Beschreibung

Die Erfindung betrifft ein Instantgranulat nach dem Oberbegriff des Anspruches 1, ein Verfahren zu seiner Herstellung, sowie die Verwendung einer Mischung wenigstens eines Soja-Materials mit wenigstens einer pharmazeutisch zulässigen Calcium-Verbindung.

Die EP-A2 398 867 beschreibt ein kakaohaltiges, ernährungsphysiologisch hochwertiges Getränk für Kinder und Jugendliche in der Wachstumsphase, das ausser dem Kakao noch Süssungsmittel, Aromastoffe, Weizenkeimlinge, Soja-Eiweiss, Calcium und Magnesium, die Vitamine B1, B2, B6 und C, sowie Lezithin als Emulgator enthält. Gegebenenfalls wird zur Förderung der Calcium-Resorption Lactose zugesetzt, die allerdings physiologisch, insbesondere in den vorgeschlagenen Mengen, unerwünscht ist.

Erfindungsgemäss wird hingegen ein Instantgranulat nach den kennzeichnenden Merkmalen des Anspruches 1 vorgeschlagen, das aufgrund seiner hohen Dosierung von Calcium-Ionen und von den wesentlichen Inhaltstoffen der Soja, nämlich der Isoflavone, bisher nicht in eine oral applizierbare Form gebracht werden konnte. Des weiteren wird erfindungsgemäss die Verwendung von Vitamin D3 oder von Natriummonofluorophosphat (im Gegensatz zu der eingangs genannten EP-A1 und zu konventionellen calciumhältigen Tabletten) zur Verbesserung der Calcium-Resorption, sowie die Verwendung eines solchen Granulates für die Zubereitung einer oral applizierbaren Lösung oder Suspension für die Behandlung und Prävention der Osteoporose vorgeschlagen.

Als Isoflavon-Donatoren kommen insbesondere konzentrierte Soja-Isoflavone der Firma CentralSoya^{®} (USA) mit 5% Isoflavonen, oder der Firma NovaSoy^{®} (USA) mit 40% Isoflavonen in Frage.

Seit einiger Zeit sind Soja-Produkte als wirkungsvolle Komponenten bei der Verhütung von Osteoporose bekannt (Journal of Nutrition, March 1995). Verschiedene Studien haben diese Annahme erhärtet; die Eßgewohnheiten in asiatischen Ländern verglichen mit denen westlicher Länder haben einen Bezug zur Entstehung von Osteoporose gezeigt.

In westlichen Ländern wird die Osteoporose mit verschiedenen Maßnahmen, insbesonders mit hohen Dosen von Calcium zusammen mit Vitamin D3 behandelt. Nun gibt es eine große Zahl wissenschaftlicher Publikationen, bei denen Sojaprotein-Pulver aufgrund ihres Gehaltes an Phytoestrogenen eine bedeutende Rolle in der Prävention von Menopausen-Symptomen und Osteoporose zeigen (Annual Review of Nutrition 1997). Aufgrund ihrer Östrogenaktivität können sie bis zu einem gewissen Grad Osteoporose verhindern. Aus einem Bericht des Royal Hospital for Women in Australien geht hervor, daß verschiedene Sojaprodukte aufgrund ihres hohen Phytoestrogengehaltes sich positiv bei der Behandlung von Osteoporose auswirken. Eine weitere Studie besagt, dass Sojabohnenproteine wirksam gegen Knochenschwund aufgrund von Hormonmangel eingesetzt werden können.

Zum Stand der Technik: Bekannt ist es (Publikation "Bone mash calcium supplementing paste"), Calcium und ein Sojamaterial in einer Paste zu verabreichen. Dabei handelt es sich um eine pastöse Masse, die vorwiegend pulverisierte Knochen von Schweinen und Rindern enthält und nur 2 - 3% Sojabohnenpulver, aber keine Hinweise zur Lösung des Problems eines calcium- und sojahältigen Instantgranulates enthält.

Ziel der Erfindung war es nun, ein Produkt zu entwickeln, um das gewünschte Sojamaterial in Pulverform zusammen mit Calciumverbindungen und gegebenenfalls Vitaminen, insbesondere Vitamin D3, Fluorverbindungen und/oder Aminosäuren in die galenische Form eines Instantgranulats zu bringen, das eine erhebliche Menge an Sojamaterial zusammen mit calciumhältigen Substanzen organischer und/oder anorganischer Natur enthält und beim Auflösen in einem Glas Wasser suspendierbar ist.

Bei der Herstellung eines Instantgranulates mußte das Problem der Granulierung des Sojamaterials sowie die Erzielung einer guten Benetzbarkeit des Granulats gelöst werden. Ein weiteres Problem war, sowohl die Calciumverbindungen als auch das Sojamaterial zu suspendieren, sodaß man eine - zumindest über 5 Minuten - stabile Suspension ohne Sedimentierung erzielen konnte.

Erfindungsgemäss ist es nun gelungen, Calcium/Soja-Instantgranulate zur Herstellung sowohl pH-neutraler Getränke als auch säurehältiger Getränke zu erzeugen, deren gemeinsames Merkmal es ist, daß die schwer zu benetzenden Sojamaterial-Pulver mit oberflächenaktiven Substanzen zusammen mit Calciumverbindungen in einer Weise granuliert werden, daß ein leicht suspendierbares Getränk entsteht, in dem die Einnahme sowohl des Sojamaterials als auch der Calciumverbindung in angenehmer und zweckmäßiger Weise miteinander verbunden wird.

Angestrebt wurde, daß eine Dosis etwa 500 bis 1200 mg Calciumionen enthält, darüber hinaus 0,1 bis 10 g Sojamaterial, und überdies können dazu noch Vitamin D3, vorzugsweise in einer Menge von 100 bis 1000, insbesondere 200 bis 800 i.E., oder Fluorverbindungen, z.B. in einer Menge von 50 bis 100 mg Natriummonofluorophosphat, für die Prävention oder Therapie von Osteoporose inkorporiert werden.

Als Sojamateriale kommen sowohl entfettete als auch fetthaltige Sojamehle in Betracht, wobei den entfetteten Sojamehlen der Vorzug zu geben ist, weiters Sojaproteinpulver, pulverisierter Sojaschrot (vollfett oder entfettet) oder Mischungen aus diesen Produkten.
Bevorzugt ist jedoch solches Sojamaterial, das reich an den zwei wichtigsten Isoflavonen, Genistein und Daidzein ist. Basierend auf dem Verzehr von Soja-Produkten in Japan beträgt eine typische Tagesdosis an Isoflavonen ungefähr 50 mg pro Person (entsprechend American Journal of Chemical Nutrition 1995). Bei Soja-Material, das reich an Isoflavonen ist und beispielsweise 40% Isoflavone enthält, ist nur mehr eine Dosierung von beispielsweise ca. 125 mg des Sojamaterials erforderlich, d.h. es brauchen nun keine großen Mengen mehr an Sojamehl verabreicht werden. Weiters werden im Handel auch Soja-Isoflavon-Konzentrate angeboten, die alle wesentlichen Isoflavone wie Daidzeine, Glyciteine und Genisteine enthalten. Auch können handelsübliche konzentrierte Soja-Proteine bzw. Soja-Proteinkonzentrate eingesetzt werden, welche die wesentlichen Isoflavone - vorzugsweise in angereicherter Form - enthalten.

Als Calciumträger kommen vornehmlich organische Calciumverbindungen in Frage, wie z.B. Calciumglycerophosphat, Tricalciumdicitrat, Monocalciumcitrat, Calciumlaevulinat, Calciummalat und andere.
Als anorganische Calciumverbindungen sind vornehmlich Calciumcarbonat, Calciumbiphosphat, Calciumchlorid, Calciumphosphat und andere geeignet.

Die Auswahl an Calcium-Verbindungen ermöglicht es, eine große Vielzahl von Getränken von sauer bis neutral mit verschiedensten Geschmacksrichtungen von fruchtig bis sahnig herzustellen. Selbstverständlich können die so entstandenen Granulate in vielfältiger Weise mit den erlaubten Süßstoffen, wie Natriumcyclamat, Saccharin-Na, Aspartam, Acesulfam gesüßt und mit Aromen versehen werden.

Weiters werden Füllstoffe zur Granulatbildung eingesetzt wie Maltodextrin (z.B. Maltrin M 700^{®}), und/oder Zuckeralkohole wie Sorbit, Mannit; es können auch Glucose, Fructose, Lactose, Saccharose, Maisstärke und/oder hydrolysierte Stärken eingesetzt werden.

Additiv zu der Kombination von Soja mit Calcium können auch Vitamine, z.B. die Vitamine des B-Komplexes, fettlösliche Vitamine wie A, D und E, sowie Folsäure, Biotin, Cyanocobalamin, Nicotinamid, Calciumpantothenat und/oder Vitamin C integriert werden. Weiters auch Spurenelemente, wie beispielsweise Mangan, Kupfer, Zink sowie auch Mineralstoffe wie Kalium, Magnesium, Eisen, etc. Darüber hinaus ist auch eine Anreicherung mit Lysin, Myo-Inosit sowie weiteren Aminosäuren, beispielsweise Argininaspartat, Valin, Isoleucin, Alanin, Cystin, Glutaminsäure, Glycin, Phenylalanin, Histidin, Threonin, Tyrosin, etc. möglich.

Die Herstellung eines Calcium/Soja-Instantgranulates mit höheren Calciumdosen wird an Hand einiger Beispiele erläutert sowie auch ein Calcium/Soja-Instantprodukt, das neben Calcium noch Vitamine, Mineralstoffe und Spurenelemente sowie einige Aminosäuren enthält, dargelegt.

Die Herstellung erfolgt am besten so, daß die Calciumverbindung zusammen mit den Füllstoffen und den gegebenenfalls zugesetzten Mineralstoffen und/oder Süßstoffen mit der Lösung eines Bindemittels befeuchtet und granuliert wird und anschließend das Sojamaterial-Pulver auf das feuchte Granulat aufgebracht und die Masse unter Rühren auf 60°C angewärmt wird, wobei durch die vorhandene Feuchtigkeit eine Angranulierung des Sojamaterials erfolgt. Die Bindemittelllösung enthält dabei als Lösungsmittel Alkohol oder eine Mischung aus Alkohol und Wasser sowie als Zusatz ein Tensid. Anschließend wird das dabei entstandene Produkt getrocknet, wobei eine Vakuumtrocknung zu bevorzugen ist; es besteht jedoch auch die Möglichkeit, das Granulat in einem Wirbelschichttrockner zu trocknen. Nach dem Trocknen ergibt sich ein rieselfähiges Granulat, in dessen Körnern die Partikel der Calciumverbindung(en) und des pulverförmigen Sojamaterials innig vermischt vorliegen. Das Granulat kann zur Erzielung einer gleichmäßigen Körnung nach Bedarf, beispielsweise auf eine Korngrösse von 1,5 bis 2,5 mm, gesiebt und in Einzeldosen verpackt werden.

Die Bindemittel, die nötig sind, um die beiden Substanzen, also das Sojamehl und die Calciumverbindung, zu granulieren, sollten bevorzugt solche Substanzen sein, die in organischen Lösungsmitteln oder Lösungsmittelgemischen mit Wasser löslich sind. Dazu gehören in erster Linie Polyvinylpyrrolidon, Polyethylenglycol 6000, so wie auch konzentrierte Lösungen von Zuckeralkoholen. Es können aber auch Saccharose, Fructose und Glucose Verwendung finden. Dabei sind - wie eingangs erwähnt - alkohollösliche Substanzen bzw. in organischen wässrigen Lösungsmittel lösliche Substanzen zu bevorzugen, weil einerseits die Granulierung mit rein wässrigen Bindemittellösungen speziell das Sojaprotein-Pulver mit seinen Wirkstoff-Prinzipien negativ beeinflussen kann und weil andererseits die Trocknung eines mit wässriger Lösung behandelten Calcium/Soja-Granulats nur erschwert möglich ist.

Als Tenside, die für die bessere Benetzbarkeit der verschiedenen Sojamaterial-Pulver bzw. für eine Verbesserung der Suspendierbarkeit des Calcium/Soja-Granulatseingesetzt werden, sind Natriumdioctylsulfosuccinat, Polysorbate, Polyoxyethylenglycerin-Fettsäureester und Natriumlaurylsulfat heranzuziehen.

Die Erfindung soll an Hand einiger Beispiele erläutert werden:

### Beispiel 1:

### Herstellung eines Calcium/Soja-Granulats mit 500 mg Calcium und 400 i.E. Vitamin D 3:

125 Gew.teile Calciumcarbonat, 25 Gew.teile Sorbitol, 37 Gew.teile Saccharose, 400 Gew.teile Maltodextrin sowie 4 Gew.teile Aspartam werden unter Erwärmen gleichmäßig unter Rühren vermischt. Die Mischung wird mit einer Lösung bestehend aus 1,5 Gew.teile Polysorbat, 16,8 Gew.teile Glucosesirup flüssig (entsprechend 13,5 fest) in 50 Gew.teilen 40%igem Ethanol benetzt und 3 Minuten gleichmäßig verteilt. 770 Gew.teile Sojamehl werden zugefügt und die Mischung unter Rühren auf 60 - 65°C erwärmt, wobei die Granulierung erfolgt. Anschließend wird das Produkt getrocknet, wobei sowohl eine Vakuumtrocknung als auch eine Bandtrocknung erfolgen kann.
Zu dem auf 1,5 - 2,0 mm gesiebten Granulat werden 400 i.E. Vitamin D3 sowie Aromen zugefügt. Eine Dosis von 14 g enthält neben 500 mg Calciumion und 400 i.E. Vitamin D3 ausserdem 7,7 g Sojamehl, entsprechend einem Gehalt von 15,4 - 23 mg Isoflavonen (laut Technical Bulletin enthält 1 Gramm Sojamehl 2 - 3 mg Isoflavone). In Wasser eingebracht und kurz gerührt ergibt dies eine milchige, angenehm schmeckende Suspension.

Wie in Beispiel 1 angeführt, können statt Sojamehl auch Sojaproteine sowie mit Isoflavonen angereichertes Sojamaterial verarbeitet werden, wobei die erforderliche Menge an Granulierlösung eine gewisse Abhängigkeit vom eingesetzten Sojamaterial hat.

Bei organischen Calciumsalzen können die Calciumsalze auch mit Sojamehl vermischt und anschließend mit einer alkoholischen oder alkoholischwässrigen Lösung mit einem Bindemittel granuliert werden.

### Beispiel 2:

### Mit Calciumglycerophosphat als Calciumverbindung:

Die Herstellung erfolgt analog den vorhergegangenen Beispielen. Es werden 10 Gew.teile pulverförmiges Soja material mit 5,2 Gew.teilen Calciumglycerophosphat (entsprechend 1000 mg Calciumion) in einem Granulator vermischt. Das Produkt wird unter Mischen auf 50°C erwärmt und mit einer Lösung bestehend aus 0,7 Gew.teilen Ethanol, 1 Gew.teil Polyethylenglycol 6000, das aufgeschmolzen wird, und 0,004 Gew.teilen Sorbitanoleat (Tween^{®}) granuliert. Das Produkt wird unter Rühren getrocknet, vorzugsweise mittels Vakuum durch ein Sieb von 1 bis 2 mm gesiebt und mit 0,7 Gew.teilen Aroma sowie nach Wunsch mit der entsprechenden Menge an Süßstoff und gegebenenfalls an Farbstoff versetzt.
Eine Dosis von 16,2 g - entsprechend 1000 mg Calcium und 20-30 mg Isoflavonen - ergibt ein gut suspendierbares Getränk.

### Beispiel 3:

### Herstellung eines Calcium/Soja-Granulats enthaltend 160 mg Calcium sowie Mineralstoffe (Magnesium und Eisen), Spurenelemente, Vitamine und Aminosäuren:

In einen Vakuumgranulator werden eingebracht: 688 Gew.teile Calciumhydrogenphosphat, 44 Gew.teile Kaliumcarbonat, 286 Gew.teile Trimagnesium-dicitrat, 21 Gew.teile Eisengluconat, 625 Gew.teile Lysin-HCl, 12,5 Gew.teile Myo-Inosit, 4000 Gew.teile Maltodextrin, 60 Gew.teile Aspartam, 200 Gew.teile Zitronensäure, 363 Gew.teile Saccharose werden homogen vermischt. Auf diese Mischung wird eine Lösung bestehend aus 15 Gew.teilen Polysorbat, 135 Gew.teilen Glucosesirup flüssig in 490 Gew.teile 40%igem Ethanol gelöst aufgebracht und gleichmäßig verteilt. Dieser befeuchteten Mischung werden 8300 Gew.teile Sojamehl zugefügt und unter Rühren auf 60°C erwärmt, wobei eine Granulierung erfolgt. Das feuchte Granulat wird mittels Vakuum unter Rühren bei einer Temperatur von 40 - 55°C getrocknet. Zum getrockneten und auf die gewünschte Korngröße gesiebten Granulat können eine Mischung von Vitaminen des B-Komplexes, Vitamin C sowie Vitamin A, D und E, weiters auch Spurenelemente, wie Mangan, Kupfer, Zink und gegebenenfalls weitere Aminosäuren zugemischt werden. Mit Orangen- oder Zitrusaromen ergibt das Produkt bei einer Dosis von 14-18 g (enthaltend 8,3 g Sojamehl bzw. 16,6 - 25 mg Isoflavone) ein gut suspendierbares, angenehm schmeckendes Getränk.

Um eine optimale Auflösung des Instantgranulats zu erreichen, wird erfindungsgemäss das Sojamaterial (z.B. Sojamehl) bevorzugt entweder mit einer löslichen organischen Calcium-Verbindung oder mit einer Mischung aus einem geeigneten Füllstoff und einer unlöslichen anorganischen Calcium-Verbindung unter Zuhilfenahme eines geeigneten Bindemittels wie z.B. Glukose-Sirup, welcher ein Netzmittel wie z. B. Polysorbat enthält, granuliert. Auf diese Weise wird sichergestellt, dass das proteinhältige Sojamaterial (z.B.Sojamehl) gut benetzt wird und nicht verklumpt und daher auch nicht den Nachteil hat, dass es sich nur schwer in einem Getränk suspendieren lässt. Der Füllstoff bzw. die löslichen Calcium-Verbindungen werden nämlich oberflächlich angelöst, das Soja-Produkt wird dort eingebaut und das Instantgranulat lässt sich dann viel besser suspendieren. Dabei hat sich der Zusatz eines Polysorbates zu einem Glucose-Sirup als Granulationslösung als sehr zweckmässig erwiesen.

## Patentansprüche

1. Instantgranulat auf der Basis wenigstens eines Isoflavon-hältigen Sojamaterials und wenigstens einer pharmazeutisch zulässigen Calcium-Verbindung, mit einem Bindemittel und einer oberflächenaktiven Substanz, **dadurch gekennzeichnet, dass** pro 500 bis 1200 mg Calciumionen der Gehalt an Sojamaterial von 0.1 bis 10 g beträgt sowie ausserdem eine oberflächenaktive Substanz aus der Gruppe Natriumdioctylsulfosuccinat, Natriumlaurylsulfat, Polysorbate und Polyoxyethylenglycerin-Fettsäureester enthalten ist.

2. Instantgranulat nach Anspruch 1, **dadurch gekennzeichnet, dass** pro 500 bis 1200 mg Calciumionen der Isoflavongehalt 10 bis 50 mg beträgt.

3. Instantgranulat nach Anspruch 1 oder 2, **dadurch gekennzeichnet, dass** die Calciumverbindung in anorganischer Form als Carbonat, Chlorid, Phosphat oder Hydrogenphosphat oder in organischer Form als Glycerophosphat, Laevulinat, Malat oder Citrat vorliegt.

4. Instantgranulat nach einem der Ansprüche 1 bis 3, **dadurch gekennzeichnet, dass** es ausserdem Vitamine, insbesondere Vitamin D3, Fluorverbindungen und/oder Aminosäuren enthält.

5. Instantgranulat nach Anspruch 4, **dadurch gekennzeichnet, dass** es pro 500 bis 1200 mg Calciumionen 100 bis 1000, vorzugsweise 200 bis 800 i.E. Vitamin D3 enthält.

6. Instantgranulat nach Anspruch 1, 2 oder 3, **dadurch gekennzeichnet, dass** es pro 500 bis 1200 mg Calciumionen 50 bis 100 mg Natriummonofluorophosphat enthält.

7. Instantgranulat nach einem der Ansprüche 1 bis 6, **dadurch gekennzeichnet, dass** es ein Bindemittel enthält, welches in einem organischen oder wasserhältigen organischen Lösungsmittel löslich ist und vorzugsweise aus folgender Gruppe gewählt ist: Polyvinylpyrrolidon, Polyethylenglycol 6000, Glucosesirup.

8. Instantgranulat nach einem der Ansprüche 1 bis 7, **dadurch gekennzeichnet, dass** es die Calciumverbindung in anorganischer Form sowie zusätzlich bis zu 40 Gew.%, vorzugsweise 5 bis 35 Gew.% wenigstens eines Füllstoffes, insbesondere aus der Gruppe Maltodextrin und Zuckeralkoholen, enthält.

9. Instantgranulat nach einem der Ansprüche 1 bis 8, **dadurch gekennzeichnet, dass** es ausserdem wenigstens eine Substanz aus der Gruppe der Aminosäuren, Vitamine, Mineralstoffe und Spurenelemente enthält.

10. Instantgranulat nach einem der Ansprüche 1 bis 9, zur Verwendung als Arzneimittel.

11. Verwendung eines gemäss einem der Ansprüche 1 bis 10 definierten Instantgranulats für die Zubereitung einer oral applizierbaren Lösung oder Suspension zur Prävention und/oder Behandlung von Osteoporose.

## Claims

1. Instant granules based on at least one isoflavone-containing soya material and at least one pharmaceutically admissible calcium compound, comprising a binder and a surface-active substance, **characterized in that** the content of soya material is 0.1 to 10 g per 500 to 1200 mg of calcium ions and in addition a surface-active substance from the group consisting of sodium dioctylsulphosuccinate, sodium laurysulphate, polysorbates and polyoxyethylene glycerol fatty acid esters is present.

2. Instant granules according to Claim 1, **characterized in that** the isoflavone content is 10 to 50 mg per 500 to 1200 mg of calcium ions.

3. Instant granules according to Claim 1 or 2, **characterized in that** the calcium compound is present in inorganic form as carbonate, chloride, phosphate or hydrogen phosphate or in organic form as glycerophosphate, laevulinate, malate or citrate.

4. Instant granules according to any of Claims 1 to 3, **characterized in that** it additionally contains the vitamins, in particular vitamin D3, fluorine compounds and/or amino acids.

5. Instant granules according to Claim 4, **characterized in that** it contains 100 to 1000, preferably 200 to 800, IU of vitamin D3 per 500 to 1200 mg of calcium ions.

6. Instant granules according to Claim 1, 2 or 3, **characterized in that** it contains 50 to 100 mg of sodium monofluorophosphate per 500 to 1200 mg of calcium ions.

7. Instant granules according to any of Claims 1 to 6, **characterized in that** it contains a binder which is soluble in an organic or water-containing organic solvent and is preferably selected from the following group: polyvinylpyrrolidone, polyethylene glycol 6000, glucose syrup.

8. Instant granules according to any of Claims 1 to 7, **characterized in that** it contains the calcium compound in inorganic form and additionally up to 40% by weight, preferably 5 to 35% by weight, of at least one filler, in particular from the group consisting of maltodextrin and sugar alcohols.

9. Instant granules according to any of Claims 1 to 8, **characterized in that** it also contains at least one substance from the group consisting of the amino acids, vitamins, minerals and trace elements.

10. Instant granules according to any of Claims 1 to 9 for use as a medicament.

11. Use of instant granules defined according to any of Claims 1 to 10 for the preparation of a solution or suspension which can be administered orally for the prevention and/or treatment of osteoporosis.

## Revendications

1. Granulés instantanés à base d'au moins un composant du soja contenant de l'isoflavone et d'au moins un composé du calcium convenant à un usage pharmaceutique, en combinaison avec un composé liant et une substance tensioactive, **caractérisés en ce que**, pour 500 à 1200 mg d'ions calcium, la teneur en composant du soja est de 0,1 à 10 g et **en ce qu'**en outre, la substance tensioactive est choisie dans le groupe constitué par le dioctylsulfosuccinate de sodium, le laurylsulfate de sodium, le polysorbate et les esters polyéthoxylés d'acides gras et de la glycérine.

2. Granulés instantanés selon la revendication 1, **caractérisés en ce que** pour 500 à 1200 mg d'ions calcium, la teneur en isoflavone est de 10 à 50 mg.

3. Granulés instantanés selon la revendication 1 ou la revendication 2, **caractérisés en ce que** le composé du calcium est présent sous une forme inorganique en tant que carbonate, chlorure, phosphate ou hydrogénophosphate ou sous une forme organique en tant que glycérophosphate, lévulinate, malate ou citrate.

4. Granulés instantanés selon une des revendications 1 à 3, **caractérisés en ce qu'**ils contiennent en plus des vitamines et, en particulier, de la vitamine D3, ainsi que des composés du fluor et / ou des acides aminés.

5. Granulés instantanés selon la revendication 4, **caractérisés en ce que** pour 500 à 1200 mg d'ions calcium, ils contiennent de 100 à 1000 et, de préférence, de 200 à 800 unités internationales de vitamine D3.

6. Granulés instantanés selon la revendication 1, la revendication 2 ou la revendication 3, **caractérisés en ce que**, pour 500 à 1200 mg d'ions calcium, ils contiennent de 50 à 100 mg de monofluorophosphate de sodium.

7. Granulés instantanés selon une des revendications 1 à 6, **caractérisés en ce qu'**ils contiennent un composé liant qui est soluble dans un milieu solvant organique ou dans un milieu solvant organique contenant de l'eau, et qui est choisi, de préférence, dans le groupe suivant : polyvinylpyrrolidone, polyéthylène glycol 6000 et sirop de glucose.

8. Granulés instantanés selon une des revendications 1 à 7, **caractérisés en ce que** le composé du calcium est présent sous une forme inorganique et **en ce qu'**ils contiennent en plus jusqu'à 40 % en poids et, de préférence, de 5 à 35 % en poids d'au moins une charge, choisie, en particulier, dans le groupe de la maltodextrine et des sucres alcools.

9. Granulés instantanés selon une des revendications 1 à 8, **caractérisés en ce qu'**ils contiennent en plus, au moins une substance choisie dans le groupe des acides aminés, des vitamines, des substances minérales et des oligo-éléments.

10. Granulés instantanés selon une des revendications 1 à 9, pour une utilisation en tant que préparation médicinale.

11. Utilisation des granulés instantanés définis dans une des revendications 1 à 10 pour la préparation d'une solution ou d'une suspension à administrer par voie orale, pour la prévention et / ou le traitement de l'ostéoporose.
